# EUROPÄISCHE PATENTSCHRIFT

(11) **EP 1 926 454 B1**
(45) Veröffentlichungstag und Bekanntmachung des Hinweises auf die Patenterteilung: **24.03.2010**
(21) Anmeldenummer: 06805809.8
(22) Anmeldetag: 22.09.2006
(51) Int. Cl.: A61F 2/16, G02B 1/02, A61L 27/00

(54) **INTRAOKULARLINSE**
INTRAOCULAR LENS
LENTILLE INTRAOCULAIRE

(30) Priorität: 23.09.2005 DE 102005045540
(43) Veröffentlichungstag der Anmeldung: 04.06.2008
(73) Patentinhaber: Dr. Schmidt Intraocularlinsen GmbH, 53757 St. Augustin (DE)
(72) Erfinder: HAMPP, Norbert, 35287 Amöneburg (DE)
(74) Vertreter: Rau, Albrecht
(86) Internationale Anmeldenummer: PCT/EP2006/009223
(87) Internationale Veröffentlichungsnummer: WO 2007/033831

(56) Entgegenhaltungen:
- WO-A2-2004/072689
- US-A1- 2005 018 310
- US-A1- 2005 099 597

## Beschreibung

Die Erfindung betrifft eine künstliche Intraokularlinse aus einem Polymermaterial, welches eine Änderung der optischen Eigenschaften der künstlichen lntraokularlinse durch Bestrahlung mit Licht erlaubt. Dadurch kann nach der Implantation die Linse exakt auf die erforderliche Sehschärfe eingestellt werden.

Der Katarakt oder der Graue Star ist ein Sammelbegriff für Erkrankungen des Auges, die mit einer Trübung der ursprünglich klaren Augenlinse einhergehen. Weltweit sind derzeit etwa 25 Millionen Menschen wegen einer Katarakt erblindet und mindestens 110 Millionen Menschen erheblich in ihrem Sehvermögen beeinträchtigt. Eine gesicherte medikamentöse Therapie zur Rückbildung der Katarakt steht zur Zeit nicht zur Verfügung. Deshalb wird eine Katarakt im Allgemeinen durch die chirurgische Entfernung der eingetrübten Augenlinse und Implantation einer künstlichen synthetischen Okularlinse (IOL) behandelt. Dieser Eingriff gehört zu den häufigsten ophtalmologischen Operationen.

Neben dem Auftreten eines sogenannten Nachstars beziehungsweise einer sekundären Katarakt ist ein häufig auftretendes Problem, dass es zumeist nicht gelingt, die zu implantierende Linse bereits vor der Operation so zu wählen, dass nach Implantation keine weitere Korrektur der Sehschärfe mehr erforderlich ist. Weiterhin werden oftmals Sehfehler, wie zum Beispiel Kurzsichtigkeit, Weitsichtigkeit oder Astigmatismus durch ungleichmäßige Wundheilung und suboptimale Positionierung der künstlichen Intraokularlinse verursacht. So gelingt es meist nicht, die postoperative Refraktion nach einer Kataraktoperation genau vorherzusagen. Dies liegt unter anderem daran, dass es sich beim Auge um ein komplexes optisches System handelt und einige zur Abschätzung benötigte Parameter nur ungenau oder gar nicht gemessen werden können, wie beispielsweise die postoperative Vorkammertiefe nach einer Katarakteroperation praktisch vor dem Eingriff nicht bestimmt werden kann. Auch biometrische Daten des zu operierenden Auges, wie beispielsweise der Hornhautradius und die Achsenlänge, sind oft nur mit relativ großen Fehlern bestimmbar. Dies hat zur Folge, dass derzeit bei etwa 83 % der Patienten ein Wert mit einer Abweichung von maximal plus/minus 1 dpt vom angestrebten Zielwert erreicht wird, während bei den übrigen eine noch weitere Abweichung erhalten wird. Diese Abweichung muss dann durch zusätzliche Sehhilfen, wie beispielsweise eine Brille oder eine Kontaktlinse ausgeglichen werden.

Aus der US 2005/0099597 A1 und der US 2005/0018310 A1 sind Intraokularlinsen bekannt, deren optische Eigenschaften durch Einstrahlen von Licht irreversibel verändert werden können.

Aus der WO 2004/072689 A2 ist eine Linse bekannt, deren Form mittels lichtempfindlicher Einsätze verändert werden kann. Die Herstellung einer derartigen Linse mit formveränderlichen Struktur-Elementen ist äußerst aufwändig und schwierig.

Eine Aufgabe der Erfindung bestand daher darin, künstliche Intraokularlinsen bereitzustellen, mit denen das angestrebte Refraktionsziel möglichst exakt erreicht werden kann, so dass zusätzliche Sehhilfen nicht mehr erforderlich sind.

Diese Aufgabe wird erfindungsgemäß gelöst durch eine künstliche Intraokularlinse, welche aus einem Polymermaterial gebildet ist, wobei das Polymermaterial photochemisch aktivierbare Gruppen enthält, so dass eine photoinduzierte Veränderung der optischen Eigenschaften der künstlichen Intraokularlinse reversibel durchgeführt werden kann.

Bei der erfindungsgemäßen Intraokularlinse können die optischen Eigenschaften, zum Beispiel der Brechungsindex, postoperativ verändert werden, so dass eine Anpassung der Linse nach der Operation möglich ist. Dadurch kann die Feinabstimmung durch Bestrahlung der künstlichen Intraokularlinse mit Licht erfolgen, und der Patient kann ohne zusätzliche Sehhilfe auskommen. Ein weiterer Vorteil der erfindungsgemäßen künstlichen Intraokularlinse besteht darin, dass keine Spezialanfertigungen von Implantatlinsen mehr erforderlich sind, sondern Standardlinsen eingesetzt werden können, die dann postoperativ hinsichtlich ihrer optischen Eigenschaften eingestellt werden. Standardmodelle könnten beispielsweise in verschiedenen Dioptrienzahlen und in verschiedenen Größen bereitgestellt werden, beispielsweise mit einer Abstufung von einer halben oder einer ganzen Dioptrien.

Weiterhin ist es bei der künstlichen Intraokularlinse möglich, die Feineinstellung nach Ende des Heilungsprozesses durchzuführen, sodass tatsächlich eine optimale Sehschärfe erreicht werden kann.

Bei der erfindungsgemäßen künstlichen Intraokularlinse ist es durch Photoinduktion, also durch Bestrahlung mit Licht, möglich, die optischen Eigenschaften zu verändern, insbesondere die optischen Abbildungseigenschaften. Dadurch kann eine Anpassung der Linse im Auge nach Implantation erfolgen. Bevorzugt werden die Brennweite der Linse oder/und asphärische Anteile verändert, um eine hohe Sehschärfe zu erzielen.

Die Brennweite der Linse kann beispielsweise durch Änderung des Brechungsindex des Linsenmaterials verändert werden.

Die erfindungsgemäße Linse wird aus einem Polymermaterial gebildet, welches vorzugsweise einige und am besten alle der folgenden Anforderungen erfüllt. Zunächst ist für ein Linsenmaterial natürlich von Bedeutung, dass es keine oder keine störende Absorption im sichtbaren Spektralbereich zeigt. Weiterhin muss das Linsenmaterial bei Körpertemperatur, also im Bereich von ca. 35 bis 45 °C formstabil sein.

Um eine technisch einfache und kommerziell günstige Verarbeitung zu ermöglichen, muss das Material aber aus der Schmelze bearbeitbar sein, d.h. die Glasübergangstemperatur beziehungsweise der Schmelzpunkt muss einerseits über der Körpertemperatur liegen, darf aber auch nicht zu hoch sein, um eine problemlose Verarbeitung zu gewährleisten.

Vorteilhaft ist weiterhin, wenn das Linsenmaterial einen möglichst hohen Brechungsindex aufweist, da dann Linsen dünner und aus weniger Material hergestellt werden können.

Weitere vorteilhafte Eigenschaften sind Flexibilität und ein hoher Wassergehalt beziehungsweise eine hohe Wasserdurchlässigkeit des Linsenmaterials. Eine flexible Linse kann beispielsweise gerollt oder gefaltet durch einen kleineren Schnitt als eine starre Linse implantiert werden, wodurch ein schonenderer Eingriff bei der Operation möglich ist. Eine hoch wasserdurchlässige beziehungsweise wasserhaltige Linse hat den Vorteil, die Diffusion von in der Augenflüssigkeit gelöstem Nährstoff nicht zu behindern.

Die erfindungsgemäße künstliche Okularlinse ist bevorzugt aus einem Polymermaterial, ausgewählt aus Acrylpolymeren, Methacrylpolymeren oder Siliconelastomeren.

Die erfindungsgemäß eingesetzten Polymermaterialien enthalten photochemisch aktivierbare Gruppen. Bei Bestrahlung der Intraokularlinse mit Licht erfolgt dadurch eine photoinduzierte Veränderung der optischen Eigenschaften der künstlichen Intraokularlinse. Eine bevorzugte Vorgehensweise ist dabei, eine photoinduzierte Veränderung des Brechungsindex des Polymermaterials. Zur Änderung des Brechungsindex werden vorteilhafterweise zwei Kohlenstoff-Kohlenstoff-Doppelbindungen unter Einwirkung von Licht in einer [2π + 2π] Cycloaddition zu einem Zyklobutanring dimerisiert. Für den Fall, dass an wenigstens einer der C-C-Doppelbindungen ein aromatisches π-System als Rest angehängt ist, nimmt die Polarisierbarkeit in Richtung der Doppelbindung stark ab, da die Resonanz mit dem π-System nach der Dimerisierung nicht mehr möglich ist. Die Dimerisierung bzw. Bildung des Cyclo-Butanrings bewirkt somit eine Verringerung des Brechungsindex. Dieser Effekt ist noch stärker für den Fall, dass an die C-C-Doppelbindung zwei aromatische π-Systeme gebunden sind, die über die dimerisierbare Doppelbindung ein konjugiertes System bilden. Andersherum kann durch Spaltung eines Cyclo-Butanrings der Brechungsindex erhöht werden.

Besonders bevorzugte photochemisch aktivierbare Gruppen sind Cumaringruppen, Chalcone, Zimtsäuregruppen und/oder Cyclobutangruppierungen.

Bevorzugt sind die photochemisch aktivierbaren Gruppen kovalent, insbesondere als Seitengruppen, an das polymere Material der Intraokularlinse gebunden. Es ist aber auch möglich, künstliche Intraokularlinsen aus einem Polymermaterial bereitzustellen, in welchem Moleküle mit photochemisch aktivierbaren Gruppen eingelagert oder eingebettet sind.

Besonders bevorzugt sind künstliche Intraokularlinsen auf Grundlage von Polymethacrylcumarinen, Polyacrylcumarinen, Polymethacryl-Zimtsäureester, Polyacrylzimtsäureester, Polyvinylzimtsäureester sowie Siliconen, an welche Cumaringruppen, Zimtsäuregruppen oder/und Cyclobutangruppen kovalent gebunden sind.

Ein besonders bevorzugtes Linsenmaterial ist Poly(7-methacryloyloxycumarin) (PMAOC). Poly(7-methacryloyloxycumarin) kann nach bekannten Verfahren hergestellt werden (siehe z.B. WO 96/10069 oder US 2,725,377). Dabei wird zunächst 7-Hydroxycumarin mit Methacrylsäurechlorid verestert und das entstehende Produkt anschließend polymerisiert.

Ein weiteres bevorzugtes Material für die erfindungsgemäßen Intraokularlinsen ist Poly(vinylzimtsäureester), welcher durch Umsetzung von Poly(vinyllalkohol) mit Zimtsäurechlorid erhalten werden kann

Ein weiteres bevorzuges Linsenmaterial ist Poly (cynamoyloxyethylmethacrylat) (PCEM). Zur Herstellung wird Hydroxethyl mit Acrylat zunächst radikalisch polymerisiert und das Reaktionsprodukt anschließend mit Zimtsäurechlorid verestert.

Die erfindungsgemäßen Linsen weisen vorteilhafterweise einen Brechungsindex n von 1,3 bis 2,0, bevorzugt von 1,5 bis 1,9, und mehr bevorzugt von 1,6 bis 1,8 auf. Die erfindungsgemäß durchführbare Änderung des Brechungsindex beträgt 0,001 bis 0,1, insbesondere von 0,005 bis 0,05, und mehr bevorzugt bis 0,03. Durch diese Änderung kann eine Änderung der Dioptrienzahl bewirkt werden, die für die Anpassung im Rahmen von medizinisch relevanten Fällen vollkommen ausreichend ist. Wird beispielsweise der Brechungsindex eines Linsenmaterials von n = 1,625 nach n' = 1,605 geändert, resultiert daraus eine Veränderung der Brennweite im Kammerwasser (bei einem angenommenen Brechungsindex in Kammerwassers von n = 1,336, einem vorderen und einem hinteren Krümmungsradius der Linse r1 und r2 = 20 mm, einer Dicke der Linse in der Mitte zu 0,8 mm) von f = 4,6 cm nach f = 5,0 cm entsprechend einer Änderung der Dioptrienzahl von 21,555 nach 20,067. In diesem Fall wird also eine Änderung der Dioptrienzahl von ca. 1,5 dpt bewirkt.

In einer weiteren bevorzugten Ausführungsform wird eine Änderung der Brennweite der Linse dadurch erzielt, dass photoinduziert eine Strukturierung der Oberfläche der künstlichen lntraokularlinse erreicht wird. Dazu werden nur bestimmte Bereiche mit photochemisch aktivierbaren Gruppen versehen oder nur bestimmte Bereiche mit Licht bestrahlt, sodass nur in diesen Bereichen eine Photoreaktion stattfindet. Bevorzugt wird dabei ein Effekt wie bei einer Frenelllinse erzielt.

In einer weiteren bevorzugten Ausführungsform wird photoinduziert eine Veränderung der Form der Intraokularlinse bewirkt, beispielsweise indem das Profil geändert wird oder die Linse elastisch durch die Photoreaktion verformt wird. Dies kann z.B. durch photoinduzierte Dichteänderungen des polymeren Linsenmaterials erzielt werden. Durch die Änderung der Dichte des Materials kann z.B. eine Änderung der Dicke der Linse in bestimmten Bereichen und damit eine Krümmungsänderung bewirkt werden.

Bei den erfindungsgemäßen Intraokularlinsen kann es sich um eine Irisgetragene oder Vorderkammerlinse handeln, bevorzugt handelt es sich aber um eine Hinterkammerlinse. Es handelt sich also um Linsen, die im Inneren des Auges implantiert werden und nicht um Linsen, die auf oder innerhalb der Hornhaut eingesetzt werden. Die Dicke der Linse beträgt üblicherweise 0,8 bis 2 mm, wobei innerhalb eines Gesamtdurchmessers von ungefähr 12 bis 13 mm ein optisch wirksamer Teil mit einem Durchmesser von ca. 5 bis 7 mm vorliegt. Die Linsen sind im Sichtbaren lichtdurchlässig, wobei eine leichte Absorption im Bereich von 400 bis 500, insbesondere 400 bis 450 nm tolerierbar ist, und insbesondere eine Absorption in diesem Bereich und in einem Bereich < 400 nm als Schutz vor UV-Strahlung sogar gewünscht sein kann.

Erfindungsgemäß kann die photoinduzierte Veränderung der optischen Eigenschaften an der implantierten Intraokularlinse durchgeführt werden. Dies ermöglicht die nachträgliche Justierung der Sehschärfe nach Implantation bzw. nach Abheilen des Auges nach einer Operation.

Erfindungsgemäß wird dabei eine Intraokularlinse bereitgestellt, welche bereits vor Implantation aus einem Polymermaterial besteht. Es findet also keine in situ Polymerisierung im Auge oder das Liefern von Monomermaterial, welches dann im Auge polymerisiert werden soll, statt. Vielmehr wird die Linse selbst bereits vorher gebildet und lediglich die optischen Eigenschaften der Linse werden durch photoinduzierte Veränderung durch eine Photoreaktion unter Verwendung von photochemisch aktivierbaren Gruppen verändert.

Die photoinduzierte Veränderung der optischen Eigenschaften erfolgt vorzugsweise durch Einstrahlen von Licht in bestimmten Spektralbereichen. In einer Ausführungsform wird Licht in einem Spektralbereich von 200 nm bis 1500 nm eingestrahlt. Bei Einstrahlen von Licht im UV-Bereich, insbesondere von 200 bis 400 nm erfolgt die photoinduzierte Veränderung bevorzugt durch eine. 1-Photonenabsorption. Die beispielsweise mittels eines Lasers oder einer Quecksilberdampflampe (ggf. unter Ausblendung bestimmter Wellenlängenbereiche) eingestrahlte_{.} Energie beträgt in diesem Fall vorzugsweise ≥ 0,05 J cm⁻², insbesondere ≥ 0,1 J cm⁻², mehr bevorzugt ≥ 0,2 J cm⁻², und am meisten bevorzugt 0,3 J cm⁻² und bis zu 10 J cm⁻², insbesondere bis zu 5 J cm⁻², mehr bevorzugt bis zu 2,5 J cm⁻², noch mehr bevorzugt bis zu 2 J cm⁻², und am meisten bevorzugt bis zu 1 J cm⁻². Die eingestrahlte Lichtenergie wird auf jeden Fall so eingestellt, dass es zwar zu einer Photoreaktion der photochemisch aktivierbaren Gruppen kommt, insbesondere zu einer Cyctobutanbildung oder -spaltung, wie oben erläutert, dass aber keine Ablation des Linsenmaterials stattfindet. Die eingestrahlte Energie kann auch in Abhängigkeit der Beladung mit photochemisch aktiven Gruppen eingestellt werden, wobei bei kovalenter Bindung die Beladung bevorzugt ≥ 50 %, ≥ 70 %, ≥ 90 %, und mehr bevorzugt ≥ 95 % des theoretischen Wertes beträgt.

Erfindungsgemäß erfolgt die Photoinduktion durch Zwei-Photonen- oder Mehrphotonenabsorption. Hierbei wird bevorzugt eine Wellenlänge im Bereich von 400 bis 1500 nm eingestrahlt. Bei der Zwei-Photonenabsorption wird vorteilhafterweise eine Energiedosis im Bereich von ≥ 2 kJ cm⁻², mehr bevorzugt ≥ 4 kJ cm⁻², und noch mehr bevorzugt ≥ 5 kJ cm⁻² eingesetzt und bis zu 20 kJ cm⁻², mehr bevorzugt bis zu 10 kJ cm⁻². Die Bestrahlung erfolgt bevorzugt gepulst mittels eines Lasers, wobei die Energiedichte pro Puls vorzugsweise ≥ 50 mJ cm⁻², mehr bevorzugt ≥ 100 mJ cm⁻² und bis zu 300 mJ cm⁻², mehr bevorzugt bis zu 200 mJ cm⁻² beträgt. Auch hier wird die Energie so gewählt, dass zwar die photochemische Reaktion ablaufen kann, aber keine Ablation des Linsenmaterials erfolgt.

Bei einer Zwei-Photonenanregung wird die Wellenlänge so gewählt, dass ein einzelnes Photon nicht ausreicht, die photochemische Aktivierung zu bewirken. Vielmehr muss ein zweites Photon einem Molekül nach Anregung zugeführt werden, um die erforderliche Energie zu erreichen. Bevorzugt wird die photochemische Reaktion durch zwei Photonen gleicher Wellenlänge induziert. Es sind jedoch oftmals auch Ausführungsformen mit zwei Photonen unterschiedlicher Wellenlänge vorteilhaft, diese erfordern jedoch einen größeren technischen Aufwand. Für eine Zwei-Photonenabsorption muss somit eine bestimmte Photonendichte vorliegen. Da Intraokularlinsen im Auge getragen werden und somit immer Licht ausgesetzt sind, ist es natürlich wesentlich, dass die photochemische Reaktion nicht durch Tages- oder Sonnenlicht ausgelöst wird, sondern erst bei einer höheren Photonendichte. Durch 2-Photonenabsorption mittels sichtbarem Licht kann auf einfache Weise Licht durch die Hornhaut zur Linse gebracht werden, wobei die photochemische Aktivierung nur bei einer höheren Photonendichte erfolgt als sie bei Tages- oder Sonnenlicht vorliegt. Durch die Absorption von zwei Photonen wird dabei eine photochemische Aktivierung mit UV-Energie erzielt, ohne dass eine unerwünschte Aktivierung durch Tageslicht stattfindet, da die Photonendichte von Tageslicht nicht für eine Zwei-Photonenanregung ausreicht.

Ein weiterer Vorteil der erfindungsgemäßen Linsen besteht darin, dass die photoinduzierten Veränderungen der optischen Eigenschaften schrittweise und/oder reversibel durchgeführt werden können. So kann durch abgestufte Energieeinstrahlung zunächst eine teilweise Änderung des Brechungsindex erzielt werden und nach vollständigem Abheilen noch eine Nachkorrektur durchgeführt werden. Weiterhin ist es auch möglich, die Sehschärfe schrittweise fein zu justieren. Weiterhin kann durch spezifische Spaltung oder Bildung von Cyclobutangruppierungen durch Einstrahlung der jeweils geeigneten Wellenlänge selektiv eine Erhöhung bzw. eine Verringerung des Brechungsindex und damit eine Veränderung in dem Bereich + Dpt bzw. - Dpt erzielt werden.

Die Erfindung betrifft weiterhin ein Verfahren zur Herstellung einer wie oben beschriebenen erfindungsgemäßen künstlichen Intraokularlinse, wobei zunächst ein polymerer Werkstoff bereitgestellt wird, welcher photochemisch aktivierbare Gruppen enthält und aus dem polymeren Werkstoff dann die Linse gebildet wird.

In einer weiteren besonders bevorzugten Ausführungsform enthält das Polymermaterial, aus dem die erfindungsgemäße künstliche Intraokularlinse gebildet ist, zumindest bereichsweise eine chemische Komponente in immobilisierter Form, aus der durch photochemische Aktivierung ein pharmazeutischer Wirkstoff freisetzbar ist. Der Wirkstoff weist z.B. eine antibiotische, entzündungshemmende antimikrobielle, antivirale, fungizide und besonders bevorzugt eine zelltoxische Wirkung auf. Bei dem Wirkstoff handelt es sich bevorzugt um ein Corticosteroid, ein nicht-steroidales entzündungshemmendes Mittel, einen Anti-Fibroblastenwachstumsfaktor oder/und einen Wirkstoff, der die proliferative Vitrioritinopartie oder die Gewebefibrose hemmt. Der Wirkstoff kann kovalent an das polymere Material gebunden sein, beispielsweise über photochemisch spaltbare Linkermoleküle, wobei hier Linkermoleküle aus der Gruppe bestehend aus Zimtsäure, Cumarin und Derivaten besonders bevorzugt sind. Der Wirkstoff oder ein Wirkstoffvorläufer kann auch in das polymere Material eingelagert oder eingebettet sein. Die Freisetzung des Wirkstoffes erfolgt durch Einstrahlen durch Licht, insbesondere durch Ein-Photonen-, und noch mehr bevorzugt durch Zwei-Photonenabsorption. Die Beladung von Linsen mit einem Wirkstoff und die photoinduzierte Freisetzung des Wirkstoffes wird beispielsweise in WO 01/35867 ausführlich beschrieben.

Durch die Beladung der erfindungsgemäßen Linsen mit einem Wirkstoff, der photoinduziert freigesetzt werden kann, kann durch Einstrahlung von Licht nicht nur die Sehschärfe nach der Implantation geregelt werden, sondern auch eine sekundäre Katarakt oder ein Nachstar durch Bestrahlung mit Licht behandelt werden.

Die Erfindung wird durch die beigefügten Figuren und die nachfolgenden Beispiele weiter erläutert.

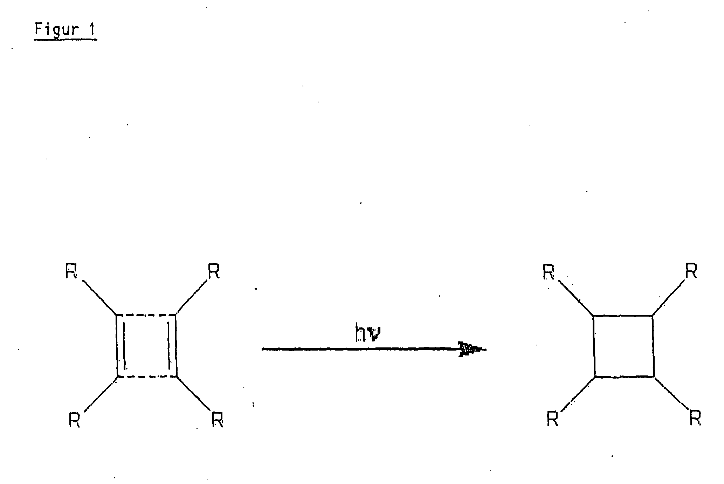

Figur 1 zeigt die schematische Darstellung der 2+2 Cycloaddition unter Bildung eines Cyclo-Butanringes.

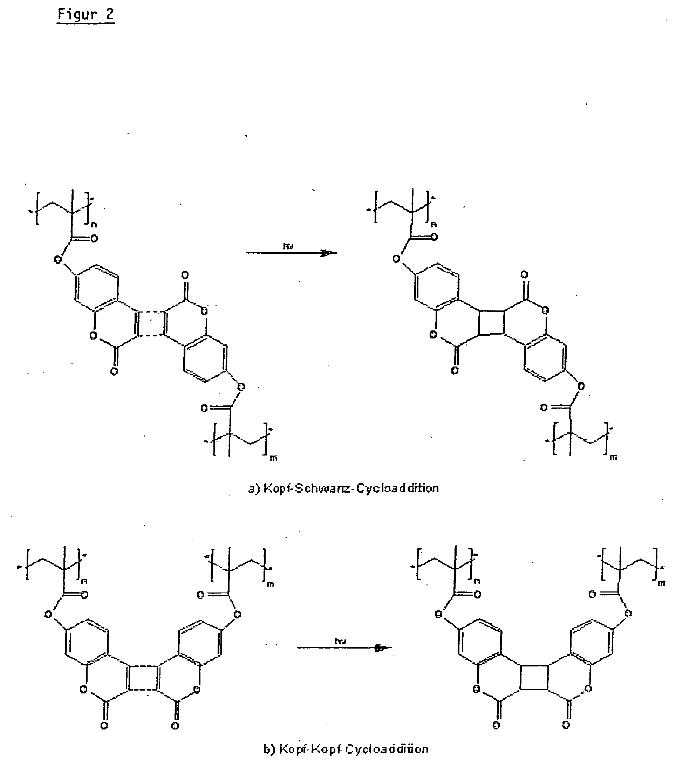

Figur 2 zeigt die Struktur von Photodimeren mit Cumarin-Seitenketten.

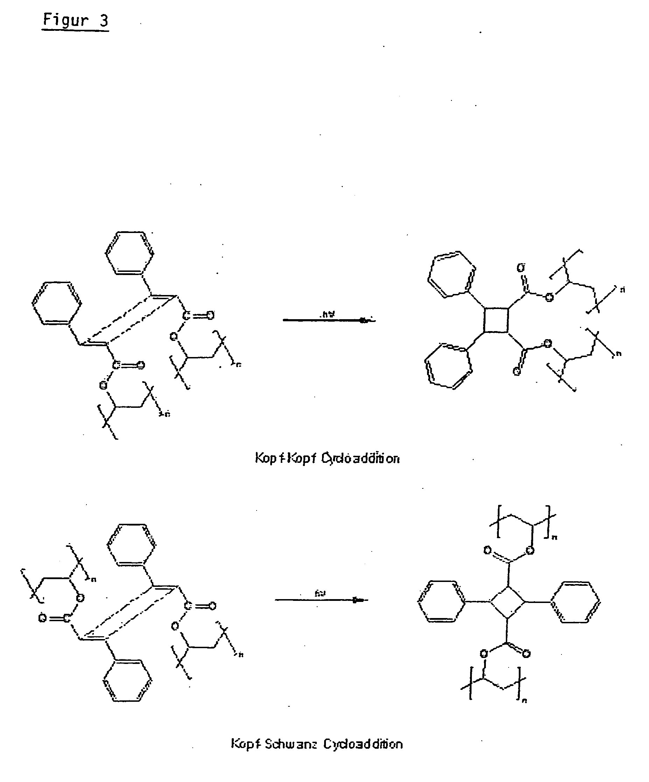

Figur 3 zeigt Strukturen von Photodimeren von Poly(vinylzimtsäureester).

### Beispiele

### Beispiel 1

### Poly(7-methacryloyloxycumarin) (PMAOC)

Zur Herstellung von Poly(7-methacryloyloxycumarin) wurde 7-Hydroxycumarin mit Methacrylsäurechlorid verestert und das Produkt dieser Reaktion anschließend radikalisch in Lösung polymerisiert. Das Polymer zeigt im sichtbaren Spektralbereich, also bei Wellenlängen zwischen 400 und 800 nm keine signifikante Absorption und erscheint daher transparent.

Zur Untersuchung der Brechungsindexänderung von PMAOC wurde zunächst mittels Spincoating ein Film aus einer Lösung von 2 Gew.-% des Polymeren in Chloroform hergestellt (2000 U/min für 20 sec). Es wurde ein Film mit einer Filmdicke von 180 +/- 4 nm erhalten. Der Film wurde mit UV-Licht der Wellenlänge 313 mm, das mittels eines Interferenzfilters aus dem Spektrum einer Quecksilberdampflampe separiert wurde, bestrahlt, und zwar bis zu einer maximalen Bestrahlungsdosis von 2,7 J cm⁻². Der Brechungsindex wurde ellipsometrich bestimmt und betrug vor der Bestrahlung 1,804 +/- 0,007. Die maximale Änderung des Brechungsindex betrug 0,034 +/- 0,009.

Aus UVNIS Spektren von PMAOC nach Bestrahlung mit unterschiedlichen Dosen einer Wellenlänge von 313 nm konnte gesehen werden, dass bei einer Energiedosis von 1,63 J cm⁻³ etwa 15 % der Cumaringruppen dimerisiert sind.

### Beispiel 2

### Poly(vinylzimtsäureester) (PVCi)

Poly(vinylzimtsäureester) kann durch Umsetzung von Poly(vinylalkohol) mit Zimtsäurechlorid gewonnen werden. Es wurde hierin ein Poly (vinylzimtsäureester) mit einem Veresterungsgrad von 95% gemäß ¹H-NMR-Daten verwendet. Das Material ist im Wesentlichen transparent, hat jedoch eine Absorption am kurzwelligen Ende des sichtbaren Spektrums bei etwa 400 bis 450 nm, weshalb das Material leicht gelblich erscheint. Dies steht einer Verwendung als Augenlinse jedoch nicht entgegen, da sich das menschliche Gehirn schnell an eine gelbliche Linse gewöhnt und die Färbung dann subjektiv nicht mehr wahrgenommen wird. Weiterhin ist der starke Anstieg der optischen Dichte unterhalb von 400 nm vorteilhaft, da das Auge dadurch vor schädlicher UV-Strahlung geschützt wird.

Zur Untersuchung der Brechungsindexänderung von PVCi wurde mittels Spincoating ein Film aus einer Lösung von 8 Gew.-% des Polymeren in Diethylenglycoldimethylester hergestellt (3000 U/min für 20 sec). Die resultierende Filmdicke betrug 292 +/- 4 nm. Der Film wurde mit UV-Licht der Wellenlänge 266 nm bis zu einer maximalen Bestrahlungsdosis von 2,8 J cm⁻² bestrahlt. Der Brechungsindex wurde ellipsometrisch bestimmt und betrug vor Beginn der Bestrahlung 1,590 +/- 0,003. Es wurde eine maximale Brechungsindexänderung Δn von 0,048 +/- 0,004 bestimmt.

Mittels Untersuchung von UVNIS Spektren konnte festgestellt werden, dass eine maximale Änderung des Brechungsindex mit einer Dimerisierung von etwa 70 % der Zimtsäuremoleküle erreicht wird.

## Patentansprüche

1. Künstliche Intraokularlinse, welche aus einem Polymermaterial gebildet ist, welches photochemisch aktivierbare Gruppen enthält, so dass eine photoinduzierte Veränderung der optischen Eigenschaften der künstlichen Intraokularlinse durchgeführt werden kann, **dadurch gekennzeichnet, dass** die photoinduzierte Veränderung der optischen Eigenschaften durch Zwei-Photonen- oder Mehr-Photonen-Absorption erfolgt.

2. Künstliche Intraokularlinse nach Anspruch 1, **dadurch gekennzeichnet, dass** es sich bei den optischen Eigenschaften, die verändert werden können, um optische Abbildungseigenschaften handelt.

3. Künstliche Intraokularlinse nach Anspruch 1 oder 2, **dadurch gekennzeichnet, dass** die Brennweite oder/und asphärische Anteile photochemisch verändert werden können.

4. Künstliche Intraokularlinse nach einem der vorhergehenden Ansprüche, **dadurch gekennzeichnet, dass** photoinduziert der Brechungsindex des Polymermaterials verändert werden kann.

5. Künstliche Intraokularlinse nach einem der Ansprüche 1 bis 3, **dadurch gekennzeichnet, dass** photoinduziert eine Strukturierung der Oberfläche der künstlichen Intraokularlinse erreicht werden kann.

6. Künstliche Intraokularlinse nach einem der Ansprüche 1 bis 5, **dadurch gekennzeichnet, dass** photoinduziert die Dichte des Polymermaterials verändert werden kann.

7. Künstliche Intraokularlinse nach einem der Ansprüche 1 bis 3 oder 6, **dadurch gekennzeichnet, dass** photoinduziert eine Veränderung der Form der Intraokularlinse erreicht werden kann.

8. Intraokularlinse nach Anspruch 7, **dadurch gekennzeichnet, dass** der Krümmungsradius einer oder beider Oberflächen verändert wird.

9. Intraokularlinse nach einem der vorhergehenden Ansprüche, **dadurch gekennzeichnet, dass** die photoinduzierte Veränderung der optischen Eigenschaften an der implantierten Intraokularlinse durchgeführt werden kann.

10. Künstliche Intraokularlinse nach einem der vorhergehenden Ansprüche, **dadurch gekennzeichnet, dass** das Linsenmaterial Cumarin-, Zimtsäure-, Chalcon- oder/und Cyclo-Butangruppierungen umfasst.

11. Künstliche Intraokularlinse nach einem der vorhergehenden Ansprüche, **dadurch gekennzeichnet, dass** die photoinduzierten Veränderungen der optischen Eigenschaften schrittweise oder/und reversibel durchgeführt werden können.

12. Künstliche Intraokularlinse nach einem der vorhergehenden Ansprüche, **dadurch gekennzeichnet, dass** photochemisch aktivierbare Gruppierungen, die eine photoinduzierte Veränderung der optischen Eigenschaften der künstlichen Intraokularlinse bewirken, kovalent an das polymere Material der Intraokularlinse gebunden sind.

13. Künstliche Intraokularlinse nach einem der vorhergehenden Ansprüche, **dadurch gekennzeichnet, dass** Moleküle mit photochemisch aktivierbaren Gruppen, welche eine photoinduzierte Veränderung der optischen Eigenschaften des Intraokularlinsenmaterials bewirken können, im polymeren Material der Linse eingebettet sind.

14. Künstliche Intraokularlinse nach einem der vorhergehenden Ansprüche, **dadurch gekennzeichnet, dass** die photoinduzierte Veränderung der optischen Eigenschaften durch Einstrahlen von Licht in einen Spektralbereich von 200 nm bis 1500 nm bewirkt wird.

15. Künstliche Intraokularlinse nach einem der vorhergehenden Ansprüche, **dadurch gekennzeichnet, dass** die photoinduzierte Veränderung der optischen Eigenschaften durch Einstrahlen von Licht mit einer Intensität von ≥ 0,1 J cm⁻² bewirkt wird.

16. Verfahren zur Herstellung einer künstlichen Intraokularlinse, wie sie in einem der Ansprüche 1 bis 15 definiert ist, umfassend die Schritte:
- Bereitstellen eines polymeren Materials, welches photochemisch aktivierbare Gruppen, die eine photoinduzierte, reversible Veränderung der optischen Eigenschaften des Linsenmaterials durch zwei-Photonen- oder Mehr-Photonen-Absorption bewirken können, enthält und
- Bilden einer künstlichen Intraokularlinse aus dem polymeren Material.

## Claims

1. Artificial intraocular lens, which is made from a polymer material containing photochemically activated groups, so that a photoinduced change in the optical properties of the artificial intraocular lenses can be carried out, **characterised in that** the photoinduced change in the optical properties is obtained by two-photon or multi-photon absorption.

2. Artificial intraocular lens according to claim 1, **characterised in that** the optical properties that can be changed are optical imaging properties.

3. Artificial intraocular lens according to claim 1 or 2, **characterised in that** the focal distance and/or aspherical portions can be changed photochemically.

4. Artificial intraocular lens according to one of the preceding claims, **characterised in that** by photoinduction the refractive index of the polymer material can be changed.

5. Artificial intraocular lens according to one of claims 1 to 3, **characterised in that** by photoinduction the surface of the artificial intraocular lenses can be structured.

6. Artificial intraocular lens according to one of claims 1 to 5, **characterised in that** by photoinduction the density of the polymer material can be changed.

7. Artificial intraocular lens according to one of claims 1 to 3 or 6, **characterised in that** by photoinduction the form of the intraocular lens can be changed.

8. Artificial intraocular lens according to claim 7, **characterised in that** the radius of curvature of one or both surfaces is changed.

9. Artificial intraocular lens according to one of the preceding claims, **characterised in that** the photoinduced change in the optical properties can be performed on the implanted intraocular lens.

10. Artificial intraocular lens according to one of the preceding claims, **characterised in that** the lens material comprises coumaric, cinnamic acid, chalcone and/or cyclobutane groupings.

11. Artificial intraocular lens according to one of the preceding claims, **characterised in that** the photoinduced changes in the optical properties can be achieved in stages and/or reversibly.

12. Artificial intraocular lens according to one of the preceding claims, **characterised in that** photochemically activated groupings, which produce a photoinduced change in the optical properties of the artificial intraocular lens, are bound covalently to the polymer material of the intraocular lens.

13. Artificial intraocular lens according to one of the preceding claims, **characterised in that** molecules with photochemcially activated groups, which can produce a photoinduced change in the optical properties of the intraocular lens material, are embedded in the polymer material of the lens.

14. Artificial intraocular lens according to one of the preceding claims, **characterised in that** the photoinduced change in the optical properties is performed by radiating light in a spectral range of 200 nm to 1,500 nm.

15. Artificial intraocular lens according to one of the preceding claims, **characterised in that** the photoinduced change in the optical properties is produced by the radiation of light with an intensity of ≥ 0.1 J cm⁻².

16. Method for producing an artificial intraocular lens, as defined in one of claims 1 to 15, comprising the steps:
- providing a polymer material, which contains photochemically activated groups, which can cause a photoinduced, reversible change in the optical properties of the lens material by two-photon or multi-photon absorption and
- forming an artificial intraocular lens from the polymer material.

## Revendications

1. Lentille intraoculaire artificielle constituée d'un matériau polymère qui contient des groupes activables photochimiquement de façon à pouvoir réaliser une modification photoinduite des propriétés optiques de la lentille oculaire artificielle, **caractérisée en ce que** la modification photoinduite des propriétés optiques s'effectue par absorption à deux photons ou à plusieurs photons.

2. Lentille intraoculaire artificielle selon la revendication 1, **caractérisée en ce que** les propriétés optiques pouvant être modifiées sont des propriétés de reproduction optiques.

3. Lentille intraoculaire artificielle selon la revendication 1 ou 2, **caractérisée en ce que** la distance focale ou/et des parties asphériques peuvent être modifiées photochimiquement.

4. Lentille intraoculaire artificielle selon l'une des revendications précédentes, **caractérisée en ce que** l'indice de réfraction du matériau polymère peut être modifié par photoinduction.

5. Lentille intraoculaire artificielle selon l'une des revendications 1 à 3, **caractérisée en ce qu'**une structuration de la surface de la lentille intraoculaire artificielle peut être obtenue par photoinduction.

6. Lentille intraoculaire artificielle selon l'une des revendications 1 à 5, **caractérisée en ce que** la densité du matériau polymère peut être modifiée par photoinduction.

7. Lentille intraoculaire artificielle selon l'une des revendications 1 à 3 ou 6, **caractérisée en ce qu'**une modification de la forme de la lentille intraoculaire peut être obtenue par photoinduction.

8. Lentille intraoculaire selon la revendication 7, **caractérisée en ce que** le rayon de courbure d'une ou des deux surfaces est modifié.

9. Lentille intraoculaire selon l'une des revendications précédentes, **caractérisée en ce que** la modification photoinduite des propriétés optiques peut être réalisée au niveau de la lentille intraoculaire implantée.

10. Lentille intraoculaire artificielle selon l'une des revendications précédentes, **caractérisée en ce que** le matériau de la lentille comprend des groupements coumarine, acide cinnamique, chalcone ou/et cyclobutane.

11. Lentille intraoculaire artificielle selon l'une des revendications précédentes, **caractérisée en ce que** les modifications photoinduites des propriétés optiques peuvent être réalisées de manière progressive ou/et réversible.

12. Lentille intraoculaire artificielle selon l'une des revendications précédentes, **caractérisée en ce que** des groupements activables photochimiquement qui opèrent une modification photoinduite des propriétés optiques de la lentille intraoculaire artificielle, sont liés de manière covalente au matériau polymère de la lentille intraoculaire.

13. Lentille intraoculaire artificielle selon l'une des revendications précédentes, **caractérisée en ce que** des molécules comportant des groupes activables photochimiquement, lesquels peuvent opérer une modification photoinduite des propriétés optiques du matériau de la lentille intraoculaire, sont noyées dans le matériau polymère de la lentille.

14. Lentille intraoculaire artificielle selon l'une des revendications précédentes, **caractérisée en ce que** la modification photoinduite des propriétés optiques est opérée par émission de lumière dans un domaine spectral de 200 nm à 1500 nm.

15. Lentille intraoculaire artificielle selon l'une des revendications précédentes, **caractérisée en ce que** la modification photoinduite des propriétés optiques est opérée par émission de lumière d'une intensité de ≥ 0,1 J cm⁻².

16. Procédé de fabrication d'une lentille intraoculaire artificielle, telle que définie dans l'une des revendications 1 à 15, comprenant les étapes suivantes :
- mise à disposition d'un matériau polymère contenant des groupes activables photochimiquement, qui peuvent opérer une modification photoinduite réversible des propriétés optiques du matériau de la lentille par absorption à deux photons ou à plusieurs photons, et
- formation d'une lentille intraoculaire artificielle à partir du matériau polymère.
